Europäisches Patentamt

European Patent Office

Office européen des brevets

(11) Numéro de publication: **0 316 280**
**A1**

## (12) DEMANDE DE BREVET EUROPEEN

(21) Numéro de dépôt: **88810759.6**

(22) Date de dépôt: **07.11.88**

(51) Int. Cl.⁴: **A 61 N 1/36**
**B 65 C 1/02**

(30) Priorité: **12.11.87 CH 4416/87**

(43) Date de publication de la demande:
**17.05.89 Bulletin 89/20**

(84) Etats contractants désignés:
**AT BE CH DE ES FR GB IT LI LU NL SE**

(71) Demandeur: **MEDICOMPEX S.A.**
**14, avenue de Sécheron**
**CH-1202 Genève (CH)**

(72) Inventeur: **Brodard, Roland**
**4 avenue des Châtaigniers**
**CH-1844 Villeneuve (CH)**

(74) Mandataire: **Kirker, Gaylord Emile et al**
**c/o KIRKER & Cie S.A. 14, Rue du Mont-Blanc Case**
**postale 872**
**CH-1211 Genève 1 (CH)**

(54) **Installation de stimulation électrique neuro-musculaire.**

(57) L'appareil programmeur (1) et l'appareil stimulateur (2) sont capables de fonctionnement autonome. Une liaison on-line (3) permet de les connecter lorsqu'ils coopèrent. L'appareil programmeur (1) sert à éditer des cartes mémoires (12) du type carte à puces à mémoire vive destinées à enregistrer des programmes de traitement complets et individualisés par patient, lorsque ces cartes sont placées dans un récepteur (11) pourvu d'un connecteur (38) destiné à coopérer avec un connecteur (37) de cette carte (12). L'appareil stimulateur (2) est prévu pour fonctionner de façon autonome sous la commande d'une telle carte-mémoire placée dans son récepteur (25). La liaison on-line permet notamment de modifier au moyen du clavier (8) le programme enregistré sur une carte-mémoire (12) et dans la mémoire (7) de l'appareil programmeur (1), en fonction des réactions du patient soumis à ce programme au moyen de l'appareil stimulateur (2), afin d'individualiser et d'adapter le programme à chaque patient.

En fonctionnement autonome de l'appareil stimulateur (2) ce dernier enregistre sur la carte-mémoire (12) qui le commande, des temps d'utilisation par le patient, à des fins de contrôle de l'observance.

FIG.1

## Description

### Installation de stimulation électrique neuro-musculaire

On connaît déjà des installations comprenant un appareil programmeur et un appareil stimulateur fonctionnant indépendamment l'un de l'autre. L'appareil programmeur est agencé pour enregistrer un programme de traitement sur un support d'informations à mémoire morte et ce support d'informations porteur du programme est placé dans l'appareil stimulateur pour le commander et faire exécuter le programme.

L'inconvénient de ce système est sa rigidité, c'est-à-dire que le programme ainsi enregistré est définitif d'emblée et ne peut pas être adapté de façon précise au cas du patient du fait de l'emploi ne mémoires mortes.

Par ailleurs, on connaît une installation comprenant un appareil programmeur et un appareil stimulateur pouvant travailler on-line. Mais dans ce cas il n'y a pas de support d'informations indépendant des deux appareils, c'est l'appareil programmeur qui permet d'enregistrer un programme établi par le thérapeute dans une mémoire vive de l'appareil stimulateur. L'inconvénient est que chaque fois que l'appareil stimulateur doit être employé par un patient différent, on doit effacer le programme qu'il a reçu antérieurement et enregistrer à nouveau le programme concernant le nouveau patient. Le premier programme une fois effacé n'a laissé de trace nulle part et lorsque le premier patient doit de nouveau être traité, il faut une fois de plus réenregistrer dans l'appareil stimulateur le programme qui lui convient. Il en résulte des lenteurs excessives dans les traitements et une utilisation irrationnelle du matériel avec la possiblité de risque d'erreurs du fait des réintroductions successives d'un même programme à des intervalles de temps plus ou moins considérables.

La présente invention vise à fournir une installation de stimulation électrique neuro-musculaire qui soit exempte de tous les défauts qui viennent d'être relevés et qui, par conséquent, permette d'une part d'établir pour chaque patient un programme parfaitement individualisé tenant compte des particularités physiologiques et pathologiques du patient. De plus, l'installation doit assurer la sécurité du patient en conditionnant le traitement par des limites imposées dans l'intensité et la durée des courants utilisés. Cette installation doit de plus permettre d'utiliser un même appareil stimulateur successivement pour des patients différents, sans perte de temps, c'est-à-dire en faisant commander cet appareil stimulateur par différents supports d'informations correspondant chacun à un patient déterminé, chacun de ces supports d'informations contenant le programme individualisé d'un patient. Ainsi, d'une part chaque programme individualisé reste à disposition en permanence sur le support d'informations et n'a pas à être recomposé par le thérapeute; d'autre part l'appareil stimulateur est immédiatement disponible lors d'un changement de patient puisqu'il suffit alors d'introduire dans cet appareil le support d'informations correspondant au patient.

L'installation de stimulation neuro-musculaire selon l'invention est conforme à la revendication 1.

Le dessin annexé représente schématiquement et à titre d'exemple une forme d'exécution de l'installation selon l'invention.

La figure 1 est un schéma-bloc de l'ensemble de l'installation.

La figure 2 représente l'appareil programmeur avec son couvercle amovible en position ouverte.

La figure 3 est une vue en plan de la partie mécanique de l'appareil programmeur qui est cachée sur la figure 2 par la carrosserie de cet appareil.

La figure 4 est une coupe longutidinale selon 4-4 de figure 3 qui montre le dispositif d'impression, d'avancement et de coupe du papier.

La figure 5 est une vue de détail correspondant à la figure 4 et montrant le mécanisme d'impression en position de travail.

La figure 6 est une vue latérale selon la flèche 6 de figure 5.

La figure 7 est une vue analogue à la figure 5 mais montrant le mécanisme d'impression en position d'introduction du papier.

La figure 8 est une vue de détail de la gauche sur figure 4.

La figure 9 est une vue latérale de la droite de figure 4.

La figure 10 est une vue de détail de la droite de figure 4 montrant le mécanisme de coupe du papier.

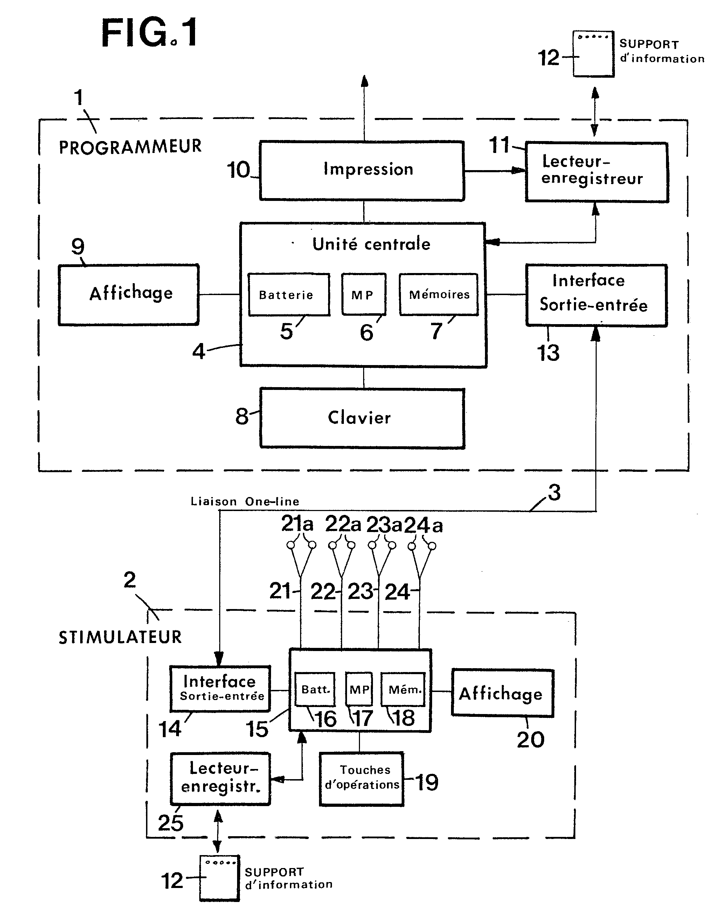

L'installation représentée sur le schéma-bloc de figure 1 comprent un appareil programmeur 1, un appareil stimulateur 2 et une liaison on-line 3 permettant de faire travailler ensemble ces deux appareils, comme on le verra dans la suite.

L'appareil programmeur comprend une unité centrale 4 dans laquelle se trouve une source de courant 5, un microprocesseur 6 et des mémoires 7 parmi lesquelles les mémoires de programme. Un clavier 8 permet à l'opérateur d'agir sur l'unité centrale 4. Cet appareil comprend encore un dispositif d'affichage 9 permettant à l'opérateur notamment de vérifier son travail sur le clavier. Cet appareil comprend en outre une unité d'impression 10 sur bande de papier autocollante, un dispositif récepteur 11 pourvu d'un connecteur et destiné à recevoir temporairement un support d'informations 12 également pourvu d'un connecteur. Ce dispositif récepteur permet de transmettre des informations de l'unité centrale 4 au support d'informations 12 et inversement. L'appareil programmeur comprend enfin un interface 13 servant à la sortie et à l'entrée d'informations par la liaison on- line 3.

L'appareil stimulateur comprend un interface 14 permettant l'échange d'informations avec l'appareil programmeur par la liaison on-line 3. Cet interface 14 est relié à l'unité centrale 15 comprenant une source de courant 16, un microprocesseur 17 et des mémoires 18. Un ensemble de touches de commande 19 est prévu pour commander les opérations de cet appareil stimulateur. Un affichage 20 est en liaison avec l'unité centrale 15.

Quatre sorties 21, 22, 23, 24 relient l'unité centrale à des paires d'électrodes 21a, 22a, 23a, 24a, destinées à être appliquées aux différents endroits du corps du patient. L'appareil stimulateur comprend enfin un dispositif récepteur 25 muni d'un connecteur et destiné à recevoir le support d'informations 12 déjà mentionné à propos de l'appareil programmeur. Ce dispositif récepteur est prévu pour coopérer dans les deux sens avec l'unité centrale 15. Le fonctionnement de l'installation représentée schématiquement sur la figure 1 est le suivant.

Pour commencer, il s'agit d'enregistrer sur le support d'informations 12 un programme de stimulation électrique individualisé, c'est-à-dire parfaitement adapté à la physiologie et à la pathologie d'un patient déterminé. A ce moment la liaison on-line 3 n'est pas nécessaire. Le thérapeute dispose d'une collection de supports d'informations tels que 12 sur lesquels différents programmes standard de stimulation sont enregistrés et qui constituent une sorte de bibliothèque de programmes. L'opérateur s'occupant maintenant d'un patient déterminé dispose un support d'informations muni du programme standard qui pourra correspondre le mieux au patient, dans le dispositif récepteur 11, puis il commande le transfert de ce programme standard dans une mémoire programme du groupe 7. Ceci fait, il remplace dans le dispositif récepteur 11 le support d'informations conte nant le programme standard par un support d'informations vierge. Ensuite, le thérapeute doit adapter ce programme standard au cas particulier du patient, d'une façon plus complète que ne pouvait le faire le programme standard. Pour cela, il agit sur le clavier 8 pour introduire les modifications et compléments au programme standard. Ces modifications et compléments sont transférés à la mémoire de programme comprise dans l'unité 7. Ceci fait, il transfère le programme standard, ainsi modifié, de la mémoire 7, par l'intermédiaire du microprocesseur 6, au support d'informations vierge 12 mis en place dans le dispositif récepteur 12.

Ceci étant fait, le thérapeute à la possiblité d'améliorer encore le programme enregistré sur le support 12, en l'adaptant davantage aux conditions particulières du patient en ce qui concerne sa réaction lors du traitement. La liaison on-line 3 est alors établie et le support d'informations 12 du patient est placé dans le dispositif récepteur 25 de l'appareil stimulateur. Les électrodes 21a, 22a, 23a, 24a sont appliquées aux endroits choisis du patient et celui-ci est soumis à la stimulation sous la commande du programme modifié une première fois, enregistré sur le support 12. Le thérapeute observe le comportement et les réactions du patient au cours des différents stades d'exécution du programme et il adapte le programme exactement au patient en procédant comme suit. Tout d'abord, en observant le patient pendant le test, il agit sur le clavier 8 pour faire enregistrer par l'intermédiaire du microprocesseur 6 les limites maximum et minimum d'intensité du courant admissibles pour ce patient, pour chacune des sorties 21, 22, 23 et 24. Ceci fait, le thérapeute a encore la possibilité d'affiner le programme en le modifiant sur certains points en fonction des réactions du patient qu'il aura observées pendant le test. La fixation des maxima et minima de courants de sortie est nécessaire pour la sécurité du patient et aussi pour l'efficacité du traitement. Toutes ces informations, c'est-à-dire maxima, minima admissibles et modifications du programme sont enregistrées simultanément sur le support 12 et dans les mémoires 7 et 18. Dès ce moment, le traitement du patient peut commencer. La liaison on-line peut alors être supprimée et c'est dès lors le support 12 qui commande l'appareil stimulateur 2. Le programme se déroule sous la commande de l'opérateur par l'intermédiaire des touches du clavier d'opération 19.

Dans ce qui a été décrit jusqu'ici, on voit que le programme appliqué au patient est établi en trois étapes : on commence par le programme standard préexistant que l'on adapte dans une certaine mesure au cas du patient en agissant sur le clavier 8 puis, dans une troisième étape, on complète et modifie encore une fois le programme en agissant sur le clavier 8 sur la base des réactions observées du patient pendant le test. En fait, la deuxième étape n'est pas indispensable et l'on peut parfaitement procéder en deux étapes, la première étant l'enregistrement d'un programme standard et dans la deuxième phase, avec la liaison on-line, on enregistre les maxima et minima de courants admissibles aux sorties et l'on fait l'adaptation du programme standard au cas particulier du patient en agissant sur le clavier 8. En ce qui concerne la bibliothèque de programmes standard, on la constitue en disposant dans chaque cas une carte vierge dans le dispositif récepteur 11 et on compose un programme standard sur le clavier 8. Une fois ce programme enregistré sur le support d'informations, celui-ci peut être retiré et constitue l'un des programmes standard. On peut aussi faire un programme standard complété ou modifié en fonction d'expériences faites ultérieurement, pour pouvoir disposer d'une carte standard supplémentaire mieux adaptée. On procédera comme suit. On place le support d'informations contenant le programme standard dans le dispositif récepteur 11. On commande par le clavier 8 le transfert de ce programme dans l'une des mémoires 7 puis on agit sur le clavier 8 pour introduire les modifications et compléments désirés du programme standard qui soit alors enregistrés dans l'une des mémoires 7. Ensuite, sous la commande du clavier, ce programmes modifié est transféré sur un support d'informations vierge que l'on a mis dans le dispositif récepteur 11 en remplacement du programme standard. Dès lors, on a un nouveau programme standard modifié à disposition dans le bibliothèque.

Les supports d'informations tels que 12 sont de préférence du type carte à puces à mémoire vive et peuvent avantageusement se présenter au format d'une carte de crédit usuelle.

L'installation décrite offre en plus de ce qui a été indiqué l'avantage de permettre au thérapeute de contrôler l'observance par le patient du programme de stimulation qu'il doit subir à l'aide de l'appareil stimulateur 2. On a vu plus haut que les valeurs maximum et minimum du courant admissible pour le patient considéré dans chacune des sorties 21, 22, 23 et 24 sont enregistrées sur le support d'informations 12 du patient. Lors de l'établissement du programme personnalisé, le thérapeute enregistre sur le support d'informations 12 individualisé, en actionnant le clavier 8, la durée prévue pour le traitement selon ce programme.

Dans ces conditions, pour vérifier l'observance de la part du patient, le thérapeute n'a qu'à placer le support d'informations 12 du patient dans le dispositif récepteur 11 de l'appareil et par le clavier 8 et par l'intermédiaire du microprocesseur 6, il questionne le support d'informations 12 qui fournit par l'affichage en 9, et éventuellement par impression sur une bande de papier en 10, le temps effectif pendant lequel l'appareil stimulateur a fonctionné lors du traitement précédent et la durée pendant laquelle, pour chacune des sorties 21, 22, 23, 24, l'intensité du courant de travail dans ces sorties était comprise entre les limites minimum et maximum programmées et enregistrées sur le support 12. Le thérapeute reçoit donc la réponse à sa question sous forme de durée. Si certaines durées manquent c'est que le patient n'a pas respecté l'observance et le thérapeute peut intervenir.

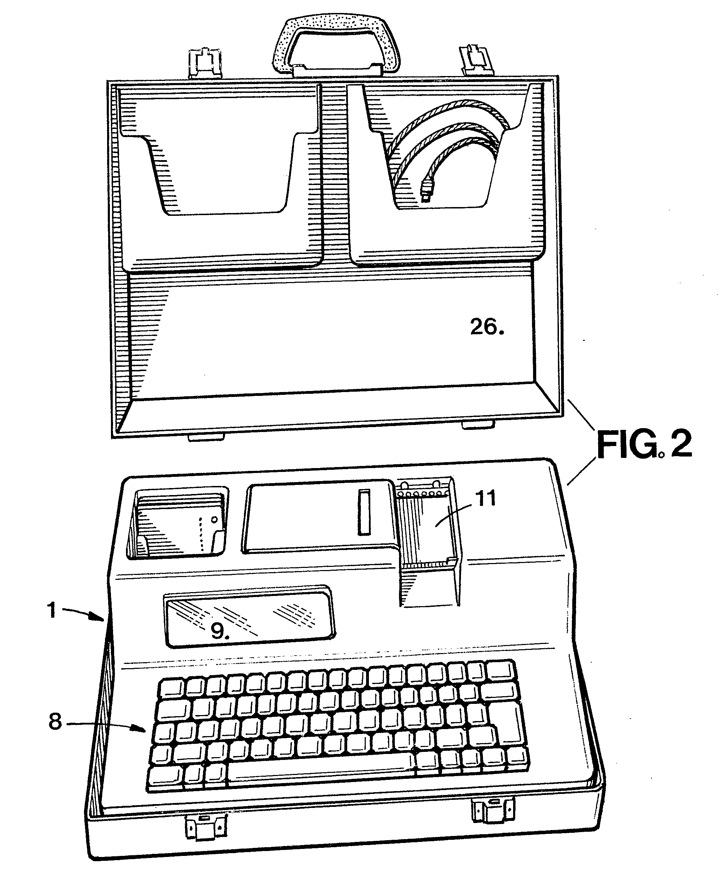

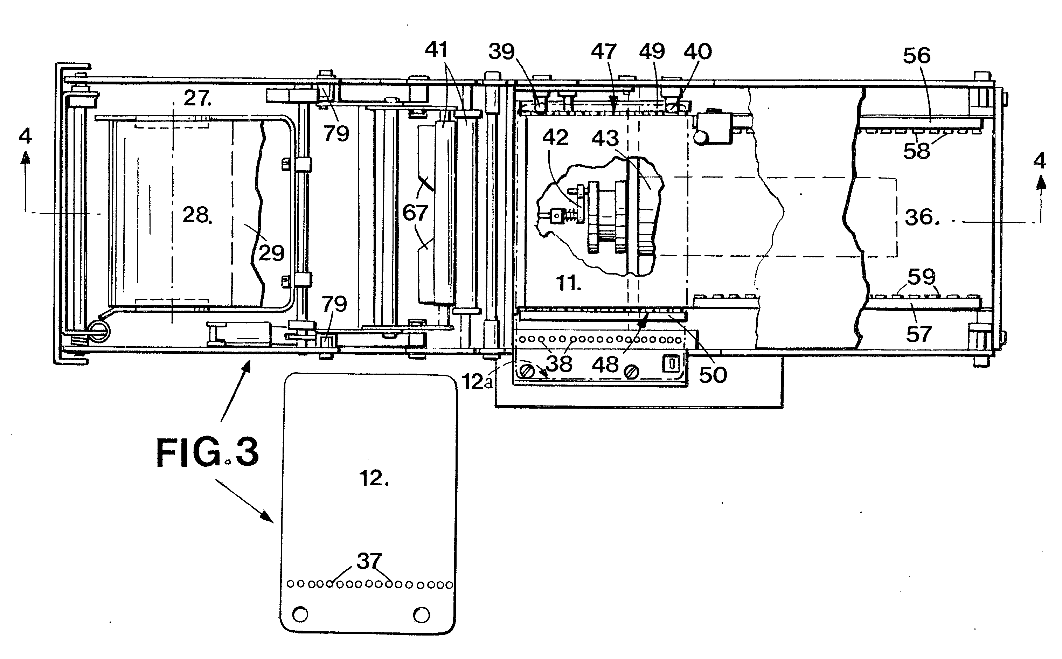

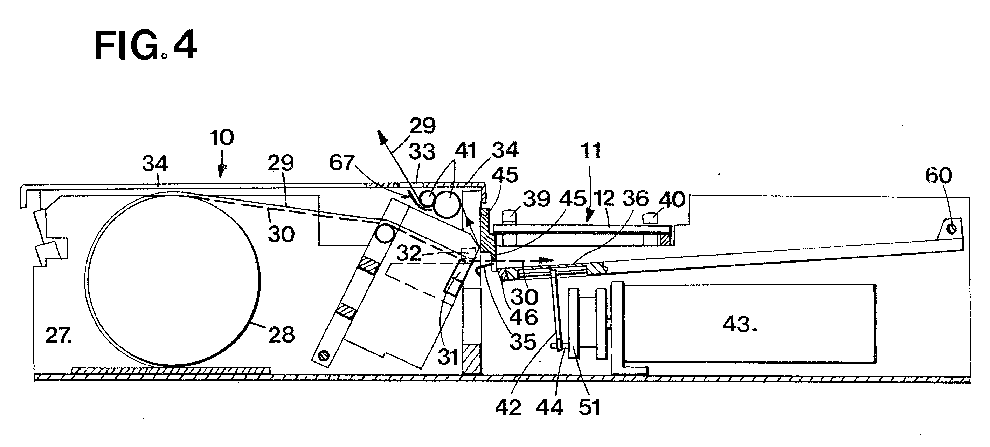

On va maintenant décrire la structure et le fonctionnement de l'unité d'impression 10 et du dispositif récepteur associé 11 faisant partie de l'appareil programmeur 1. Sur la figure 2, on voit que l'appareil programmeur 1 se présente sous forme d'une mallette dont le couvercle 26 est détachable. Sur les figures 3 et 4, on voit le dispositif d'impression sur une bande de papier autocollante, avec en plus les moyens assurant la séparation de la bande de papier imprimée de la bande lui servant de support, ainsi que la coupe au format de la bande imprimée ainsi séparée de son support et le collage de la portion de bande coupée sur le support d'informations 12. Tous ces éléments sont disposés dans un bâti 27. On voit en 28 un rouleau de papier alimentant le dispositif d'impression. On a représenté en trait plein, en 29, la bande support et en trait pointillé 30 la bande de papier destinée à recevoir l'impression. La raison d'être du support 29 est qu'il s'agit d'une bande 30 autocollante. L'ensemble des bandes 29 et 30 venant du rouleau 28 pénètre dans l'imprimante où elles passent ensemble entre la tête d'impression 31 et une enclume 32, après quoi les bandes 29 et 30 sont séparées, comme on le verra plus loin, et la bande support 29 sort à travers une fente 33 d'un couvercle 34 du mécanisme d'impression. La bande imprimée 30 arrive au contact d'un guide fixe 35 qui l'oblige à passer entre les deux lames ouvertes d'un ciseau , comme on le verra par la suite, et arriver au-dessus d'une table oscillante 36 disposée au-dessous du dispositif récepteur 11. On voit un support d'informations 12 disposé dans ce récepteur 11.

Sur la figure 3, on voit le support d'informations 12 en forme de carte de crédit avec on connecteur 37 et on voit en 38 le connecteur du dispositif récepteur 11. On a représentée en trait mixte, en 12a, un support d'informations 12 à la place qu'il occupe dans le dispositif récepteur 11. Le maintien en place du support 12 dans le dispositif récepteur 11 a lieu d'une part par la coopération des connecteurs 37, 38 et par l'action de deux ressorts de retenue 39, 40. Pour assurer la coupe d'une section de bande 30 à la dimension du support 12, le microprocesseur 6 commande un dispositif d'entraînement 41 de la bande 29. La séparation entre les bandes 29 et 30 se fait automatiquement en raison du petit angle entre le point d'arrivée et le point de sortie de la bande 29 entraînée par 41. La bande 30, elle continue horizontalement en étant commandée par 35. Lorsque la bande 30 a avancé de la quantité voulue, c'est-à-dire de la dimension du support 12, la table 36 commandée par la biellette 42 actionnée par un moteur 42 par l'intermédiaire d'une manivelle 44, oscille vers le haut. Cette bande 30 passe dans l'intervalle entre une lame fixe 45 d'une paire de ciseaux dont l'autre lame 46 est portée par la table 36. Le soulèvement de la table 36 produit la coupe du papier. La surface supérieure de la section de bande coupée est celle qui est autocollante et le mouvement de soulèvement de la table 36 se poursuit jusqu'à ce que la section coupée vienne s'appliquer contre la face inférieure du support 12. La poursuite du mouvement ascendant de la table 36 provoque l'extraction du support 12 hors du dispositif récepteur 11 en détachant ce support du connecteur 38 et en faisant écarter les ressorts 39, 40.

La longueur de la table 36 correspond à la longueur des sections de bande 30 sur lesquelles, comme on l'a vu précédemment, on imprime le programme complet détaillé enregistré par ailleurs sur le support d'informations 12. Il peut d'ailleurs y avoir plusieurs sections de bande pour contenir, sous forme écrite en langage clair, l'ensemble d'un programme.

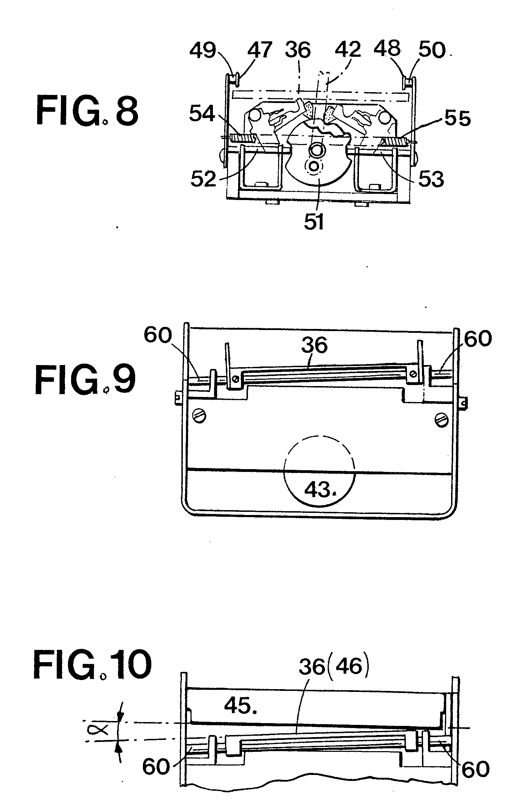

Il est nécessaire d'assurer que la section de bande 30, qui avance le long de la table 36 est qui est encollée sur sa face supérieure, reste bien en place sur cette table et ne risque pas de se déformer et d'adhérer quelque part en produisant un bourrage. A cet effet, il est prévu les moyens suivants. Sur la longueur de la table 36 qui correspond à la longueur d'une section de bande 30 devant se coller sur le support d'informations 12, il est prévu deux rangées de galets 47, 48 portées chacune par une pièce rectiligne 49, respectivement 50, parallèle à la direction générale longitudinale de la table 36. Les barres 49 et 50 sont déplaçables comme on va l'expliquer en liaison avec la figure 8, pour pouvoir être écartées l'une e l'autre d'une certaine quantité. En position normale, les galets 47, 48 se trouvent au-dessus des bords longitudinaux de la table 36, à très faible distance de celle-ci, distance juste suffisante pour laisser passer entre ces galets et cette table la portion de bande 30. En d'autres termes, ces galets 47, 48 maintiennent la bande à plat sur la table 36.

Sur la figure 8, les barres 49, 50 sont représentées en position normale de rapprochement maximum. Sur la figure 3, par contre, ces barres sont représentées en position écartée où les galets 47, 48 se trouvent au-delà des bords longitudinaux de la section de bande 30. Ceci est prévu pour laisser le passage à la table 36 dans son mouvement vers le haut qui assure la coupe du papier, l'application de la section de bande 30 coupée et l'éjection du support d'informations 12.

On a indiqué plus haut comment se fait le mouvement de la table 36. Le mouvement d'écartement des barres 49, 50 se fait lui aussi à partir du moteur 43 par l'intermédiaire d'une came 51 entraînée par le moteur 43, came qui agit sur deux tiges coulissantes coaxiales 52, 53, sollicitées par des ressorts de rappel 54, respectivement 55. La rotation de la came oblige les tiges 52, 53 à s'écarter l'une de l'autre, ce qui provoque l'écartement des barres 49, 50. Les ressorts de rappel 54, 55 ramènent automatiquement ces barres en position normale. Il est clair que la came 51 et la biellette 42 agissent en synchronisme, étant entraînées par le même moteur, ce qui assure que les barres 49, 50 et les galets qu'elles supportent s'écartent juste avant que la table 36 ne commence à monter et que ces barres ne reviennent en position normale qu'après que la table 36 soit retournée en position inférieure.

La partie de la table 36 située au-delà du dispositif récepteur 11 est munie de deux barres fixées sur elle, 56, 57, qui sont dans le prolongement exact des barres 49, 50 lorsque celles-ci se trouvent en position normale. Ces barres 56, 57 sont munies chacune d'une série de galets 58, 59 pareils aux galets 47, 48 et remplissant la même fonction de maintien de la bande de papier 30 en application sur la table 36 jusqu'au moment où l'extrémité libre de cette bande atteint l'extrémité de la table. Pour simplifier, sur la figure 4, on n'a pas représenté les barres 49, 50, 56 et 57, de même que les galets qu'elles portent.

En ce qui concerne la coupe de la bande de papier, il est prévu, comme le montrent les figures 9 et 10, une inclinaison fixe de la table 36 dans le sens transversal. Sur les figures 9 et 10, on voit en 60 l'axe fixe légèrement oblique, autour duquel la table 36 oscille sous la commande de la biellette 42. On voit en α l'angle que fait cet axe et, par conséquent, la table 36 par rapport à l'horizontale qui coïncide ici avec l'arête tranchant du couteau 45. Le mouvement d'oscillation vers le haut de la table 36 fait que les lames 45 et 46 agissent à la façon d'une paire de ciseaux.

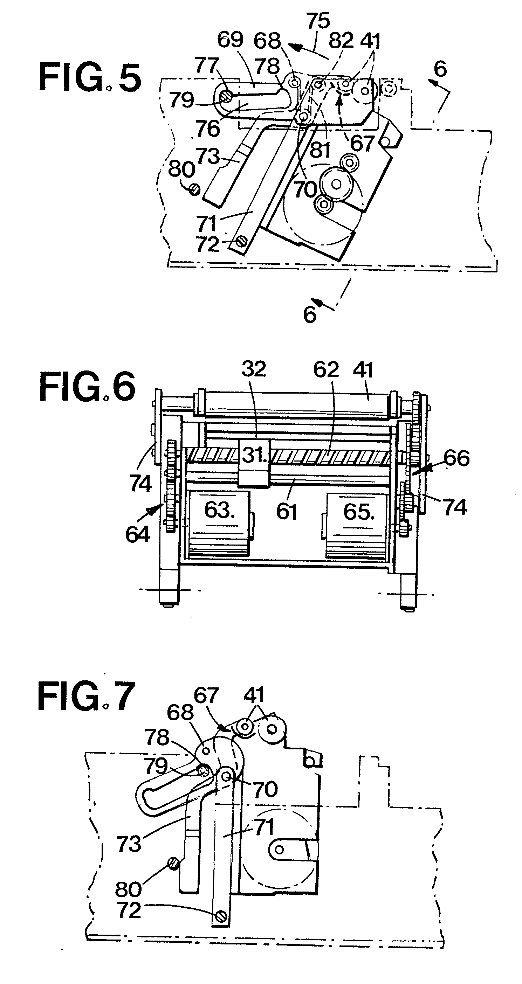

En ce qui concerne le mécanisme de l'impression proprement dit, il est d'un type connu et on se bornera à décrire ce qui suit en référence aux figures 5, 6 et 7. La tête d'impression 31 est une tête d'impression thermique montée coulissante sur une tige fixe transversale 61 pour se déplacer le long de cette tige sous la commande d'une vis 62 dont l'axe est parallèle à celui de la tige 61. Le mouvement de va-et-vient de la tête d'impression 31 est commandé par un moteur pas-à-pas 63, par l'intermédiaire d'un train d'engrenage 64. La tête d'impression 31 est appliquée par un ressort non représenté sur l'enclume 32 (figures 4 et 6), ou plus exactement sur l'ensemble des bandes 29 et 30 passant entre cette tête et cette enclume.

L'entraînement du papier (29, 30) est produit par un moteur pas-à-pas 65, par l'intermédiaire d'une démultiplication 66 qui entraîne les rouleaux 41. Le papier qui quitte le second des rouleaux 41 (qui n'est pas moteur) pour passer dans l'ouverture 33, est guidé par un guide fixe 67. La figure 5 représente les organes du mécanisme d'impression en position de travail, tandis que le figure 7 montre ces mêmes organes en position de mise en place du papier entre les deux rouleaux 41, c'est-à-dire en position écartée de ces deux rouleaux l'un de l'autre. Le passage de l'une de ces positions à l'autre a lieu en saisissant une tige transversale 68 solidaire à ses deux extrémités d'une paire de pièces de verrouillage 69. Ces pièces 69 sont articulées en 70 sur un levier 71 oscillant autour d'un axe fixe 72. Autour de l'axe 70 peut osciller une paire de leviers 73 portant à leur extrémité supérieure le rouleau de serrage du papier appartenant au groupe 41. Le rouleau moteur du groupe 41 tourne dans deux joues latérales 74 du dispositif qui, pour simplifier, n'ont pas été représentées sur les figures 5 et 7. On voit qu'en saisissant la tige 68 pour la faire osciller dans le sens de la flèche 75, on provoque les effets suivants. Chacune des pièces 69 qui présente une découpe rectiligne 76 munie à ses extrémités de deux encoches 77, 78, se déplace de la position selon figure 5 vers celle selon figure 7. Sur la figure 5, l'encoche 77 coopère avec deux tiges coaxiales transversales 79, tandis que dans la position selon figure 7 ces tiges 79 coopèrent avec l'encoche 78. De plus, le levier 71 grâce à son articulation 70 sur la pièce 69 est entraîné par celle-ci pour tourner autour de 72 et atteindre la position selon figure 7. Simultanément, le levier 73, du fait aussi de son articulation en 70 est entraîné par la pièce 69 et vient dans la position que montre la figure 7. Une butée fixe 80 arrête toutefois le levier 73 lors de sa course, ce qui a pour effet d'écarter le rouleau de serrage du groupe 41 du rouleau moteur de ce groupe, comme on le voit sur la figure 7. Cet arrêt et cet écartement des deux rouleaux 41 sont rendus possibles par un ressort double 81 monté autour de 70 et agissant par ses deux extrémités respectivement sur 68 et sur un axe 82 du levier 73 tendant à écarter ces deux axes l'un de l'autre. Le ressort 81 a deux effets : d'une part il tend à appliquer le rouleau libre du groupe 41 contre le rouleau moteur et, d'autre part, il tend à appliquer l'encoche 77 sur 79. C'est ce ressort 81 qui, en cédant lors du mouvement de la position selon figure 5 à celle selon figure 7, permet au levier 73 d'être arrêté par 80 en cours de mouvement et, par conséquent, d'assurer l'écartement du rouleau.

L'installation décrite présente un certain nombre d'avantages que l'on peut résumer de la façon suivante. Tout d'abord, le collage automatique sur le support de données lui-même, alors qu'il est encore dans l'appareil programmeur, des données d'identification du patient remédie radicalement à toute possibilité d'erreur d'identification du support. La façon dont l'appareil programmeur et l'appareil stimulateur sont agencés pour travailler on-line permet, grâce à l'emploi de support d'informations du type carte à puces à mémoire vive, de conserver les supports d'informations en libérant l'appareil de stimulation après l'emploi par chaque patient, tout en éliminant la nécessité de réenregistrer un programme déjà utilisé. De plus, l'utilisation de mémoires vives dans le support d'informations permet la modification et le complément à volonté du programme que l'on enregistre sur le support d'informations, ce qui remédie au défaut des systèmes à mémoires mortes. Enfin, la possiblité est offerte par l'installation, comme décrit plus haut, de permettre au thérapeute de vérifier si le patient a bien respecté l'observance et de déceler, le cas échéant, s'il a triché.

**Revendications**

1. Installation de stimulation électrique neuro-musculaire, comprenant, d'une part, un appareil programmeur comportant un clavier alphanumérique de commande, un écran d'affichage, un microprocesseur et une mémoire de programme, et, d'autre part, au moins un appareil stimulateur électrique prévu pour travailler on-line avec l'appareil programmeur, au moins pendant une phase de la programmation, et pour travailler de façon autonome lors du traitement d'un patient, caractérisée en ce que l'appareil programmeur (1) comporte un dispositif récepteur (11) d'un support d'informations programmable (12), dispositif muni d'un connecteur (38) prévu pour coopérer avec un connecteur correspondant (37) que présente ce support d'informations (12), lorsqu'il est placé dans ce dispositif (11), ce support étant destiné à recevoir le programme de traitement d'un patient, de façon complète et individualisée, et à commander l'appareil stimulateur (2) pendant la phase de traitement du patient, en ce que l'appareil stimulateur (2) comporte un dispositif récepteur (25) d'un tel support d'informations (12) préalablement programmé sur l'appareil programmeur (1), selon une première version générale, du programme individualisé destiné à un patient déterminé, ce dispositif récepteur (25) étant muni d'un connecteur électrique prévu pour coopérer avec celui (37) de ce support, en ce que la liaison on-line (3), lorsqu'elle est établie, est agencée pour assurer la liaison électrique entre le clavier (8) de l'appareil programmeur (1) et ledit dispositif récepteur (25) de l'appareil stimulateur (2), pour assurer la modification, par action sur le clavier (8) de l'appareil programmeur (1), de la première version du programme sur le support d'informations (12) placé dans le dispositif récepteur (25) de l'appareil stimulateur (2), et ceci par l'opérateur, en fonction de particularités individuelles du patient et de ses réactions pendant un test de stimulation au moyen de l'appareil stimulateur (2), afin que le programme ainsi modifié soit complètement individualisé et adapté au cas du patient, des moyens (6, 17) étant prévus pour modifier simultanément et de même façon le programme dans la mémoire de programme (7) de l'appareil programmeur (1), le support d'informations (12) étant dès lors apte à commander l'appareil stimulateur (2) en fonctionnement autonome pour le traitement du patient correspondant à ce support d'informations (12) momentanément en place dans le dispositif récepteur (25) de l'appareil stimulateur (2).

2. Installation selon la revendication 1, caractérisée par des moyens (11, 6, 8) pour transférer dans la mémoire (7) de l'appareil programmeur (1), un programme standard préalablement enregistré sur un premier support d'informations (12) et convenant en première approximation à un patient donné, lorsque ce premier support (12) est placé dans le dispositif récepteur (11) de cet appareil programmeur (1), des moyens (8, 6, 11) étant prévus pour transférer sur un second support d'informations (12) placé ensuite dans ce dispositif récepteur (11) en remplacement du premier, et pour transférer sur ce second support (12), le programme standard ainsi enregistré dans la mémoire (7) de l'appareil programmeur (1), après quoi ce programme en première approximation ainsi enregistré sur le second support d'informations (12) est modifiable, par action sur le clavier (8), simultanément sur ce support d'informations (12) et dans la mémoire de programme (7), pour l'individualiser au moins partiellement au cas d'un patient donné.

3. Installation selon la revendication 2, caractérisée en ce que les appareils programmeur (1) et stimulateur (2) sont agencés pour assurer une modification supplémentaire, simultanément sur le support (12) et dans la mémoire (7) de l'appareil programmeur, du programme déjà modifié dans l'appareil programmeur (1), lorsque le support d'information (12) est placé dans le dispositif récepteur (25) de l'appareil stimulateur (2) et lorsque la liaison on-line (3) est établie entre les deux appareils (1, 2), pour affiner le programme et l'individualiser complètement en fonction des réactions du patient pendant le test de stimulation au moyen de l'appareil stimulateur (2).

4. Installation selon la revendication 2 ou 3, caractérisée en ce que l'appareil stimulateur (2) comporte, pour contrôler l'observance par le patient, des moyens (17,18,25) pour enregistrer sur le support d'informations (12) placé dans le dispositif récepteur (25) de l'appareil stimulateur (2), pendant le traitement lors du fonctionnement autonome de cet appareil (2), la durée pendant laquelle cet appareil stimulateur (2) fonctionne et la durée pendant laquelle, pour chacune des ses sorties (21, 22, 23, 24) avec électrodes (21a, 22a, 23a, 24a) d'application au patient que cet appareil comporte, l'intensité du courant de travail est comprise entre des limites minimum et maximum programmées et enregistrées sur ce support d'informations (12).

5. Installation selon la revendication 1 ou 2, caractérisée en ce que l'appareil programmeur (1) comporte un dispositif d'impression (10), pour imprimer sur une partie d'une bande auto-collante (30), au moins les données d'identification du patient, lorsque la programmation individualisée est terminée et enregistrée sur le support d'informations (12), alors que ce support est placé dans le dispositif récepteur (11) de l'appareil programmeur (1), et en ce qu'il comporte un dispositif (45, 46, 36) pour couper, appliquer et coller automatiquement ladite partie de bande auto-collante imprimée sur le support d'informations (12) se trouvant dans ce dispositif récepteur (11), et pour éjecter ensuite ce support ainsi muni de cette partie imprimée identifiant le patient auquel correspondant ce support.

6. Installation selon la revendication 5, caractérisée en ce que l'appareil programmeur (1) comporte des moyens (6,7) pour actionner le dispositif imprimeur (10), pour lui faire imprimer, en clair et de façon complète, sur au moins une autre portion de bande autocollante (30), sous la commande de la mémoire (7), le programme individualisé tel qu'enregistré dans cette mémoire (7) et en plus les données d'identification du patient.

7. Installation selon la revendication 5, caractérisée en ce que le dispositif d'impression (10) de l'appareil programmeur (1) comporte des moyens mécaniques (41, 32) pour séparer la portion imprimée de la bande autocollante (30) de son support (29), et pour la couper au format du support d'informations (12), d'une part, et à un autre format, d'autre part, et pour appliquer et coller la portion coupée au format du support (12) sur ce support, avant que les moyens d'injection de ce support produisent cette éjection.

8. Installation selon l'une des revendications précédentes, caractérisée en ce que le support d'informations (12) est du type carte à puces à mémoire vive et se présente au moins approximativement au format d'une carte de crédit usuelle.

# FIG.1

26.

**FIG. 2**

11

1

9.

8

**FIG. 3**

EP 0 316 280 A1

FIG. 4

FIG. 5

FIG. 6

FIG. 7

EP 0 316 280 A1

**FIG. 8**

**FIG. 9**

**FIG. 10**

Office européen
des brevets

RAPPORT DE RECHERCHE EUROPEENNE

Numero de la demande

EP  88 81 0759

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 197 889  (BBC-SECHERON S.A.) <br> * Colonne 5, ligne 49 - colonne 16, ligne 42; figures 1-10 * | 1-4,8 | A 61 N    1/36 <br> B 65 C    1/02 |
| A | | 5-7 | |
| A | DE-A-3 207 050  (SIEMENS) <br> * Page 4, ligne 5 - page 13, ligne 20; figures 1-3 * | 1-4,8 | |
| A | EP-A-0 201 271  (BIOMEDICAL RESEARCH LTD) <br> * Colonne 4, ligne 13 - colonne 17, ligne 44; figures 1-5 * | 1-4 | |
| A | EP-A-0 165 049  (EMPI) <br> * Page 6, ligne 23 - page 22, ligne 8; figures 1-3 * | 1-4,8 | |
| A | US-A-4 448 631  (EATON) <br> * Colonne 2, ligne 51 - colonne 11, ligne 40; figures 1-6 * | 5-7 | |
| A | US-A-4 104 105  (RAYFIELD) <br> * Colonne 2, ligne 22 - colonne 4, ligne 45; figures 1-5 * | 5-7 | |

DOMAINES TECHNIQUES RECHERCHES (Int. Cl.4)

A 61 N
B 65 C
B 42 D

Le présent rapport a été établi pour toutes les revendications

| Lieu de la recherche | Date d'achèvement de la recherche | Examinateur |
|---|---|---|
| LA HAYE | 16-02-1989 | SCHMIERER U.J. |

EPO FORM 1503 03.82 (P0402)